# EUROPEAN PATENT SPECIFICATION

(11) **EP 1 527 789 B1**
(45) Date of publication and mention of the grant of the patent: **20.03.2013**
(21) Application number: 04025295.9
(22) Date of filing: 25.10.2004
(51) Int. Cl.: A61L 15/58, A61L 24/06, A61F 5/443, A61L 24/00, A61F 5/44, A61L 28/00

(54) **Multi-adhesive medical appliance**
Multihaftende medizinische Vorrichtung
Dispositif médical multiadhérent

(30) Priority: 30.10.2003 US 515474 P
(43) Date of publication of application: 04.05.2005
(73) Proprietor: ConvaTec Technologies Inc., Las Vegas, NV 89169-6754 (US)
(72) Inventor: Stoyer, Brian C., Langhorne Pennsylvania 19047 (US); Armour, Paul, Belle Mead New Jersey 08502 (US)
(74) Representative: Mays, Julie

(56) References cited:
- EP-A- 0 413 250
- EP-A- 0 686 381
- US-A- 4 538 603
- US-A- 4 775 374

## Description

### FIELD OF THE INVENTION

The present invention relates to an adhesive medical appliance designed to be removably mounted on the skin as part of an ostomy device or wound care product and more particularly to such an appliance which has the attributes of two or more types of pressure sensitive adhesives, one of which has excellent initial wet tack characteristics and can be painlessly removed from the skin, and the other of which is flexible and comfortable to wear, exhibits minimal cold flow, can withstand high moisture conditions without disintegrating, has the ability to swell to create a seal around the stoma in a controlled fashion and can be sterilized without significant color change.

### BACKGROUND OF THE INVENTION

Ostomy devices and wound care products such as bandages must adhere to and be removable from the skin proximate the stoma or other open wound. Accordingly, adhesives with special attributes that make them suitable for use on the skin proximate the stoma or other open wound have been developed for such medical appliances.

Certain of these adhesives are skin friendly, having the capability of transferring moisture such as perspiration and wound exudate away from the skin. They have excellent initial wet tack qualities and permit non-painful removal of the appliance, even after short wear times. They are formed mainly of natural ingredients and have cold flow characteristics that permit the adhesive to "fill in" any skin irregularities, providing better adhesion to the skin.

One such skin friendly adhesive material that has been found to work particularly well is a pressure sensitive polyisobutylene (PIB) adhesive having intimately dispersed therein a water soluble or swellable hydrocolloid or mixture of hydrocolloids. That material is disclosed in U.S. Patent No. 3,972,328, issued August 3, 1976 to James Chen, and owned by the Bristol-Myers Squibb Company, Lawrenceville, New Jersey. It is commercially used on many medical products sold by the ConvaTec Division of Bristol-Myers Squibb Company.

However, the cold flow properties of the adhesive disclosed in the aforementioned Chen patent may permit flow to the point of creating undermining liquid flow channels, sometimes causing the adhesive to disintegrate when coming in contact with liquid from the stoma or wound. Thicker versions of appliances with that adhesive tend to be somewhat rigid and hence may be less comfortable to wear. That adhesive may be susceptible to absorbing liquid, drying out and subsequently creating hard crystallized edges over time.

Further, the adhesive disclosed in the Chen patent does not have the ability to swell creating a seal around the stoma. It may also exhibit some negative appearance characteristics after sterilization. After gamma irradiation, it has a propensity to excessively cold flow, discolor and, with time, become rigid and brittle.

For those reasons, other adhesive materials have been developed for use in medical appliances of this type. Those adhesives tend to be more flexible and thus more comfortable to wear. They are more moisture tolerant and have the ability to swell to create a seal around the stoma in a controlled fashion, minimizing effluent contact with the persistomal skin. Those adhesives also exhibit less cold flow and minimal product erosion when exposed to effluent. Further, the adhesives exhibit little color or cold flow after gamma sterilization.

Such adhesive materials are disclosed in U.S. Patent No. 4,551,490 issued Nov. 5, 1985 to Doyle et al., U.S. Patent No. 4,538,603, issued September 3, 1985 to Pawelchak et al., U.S. Patent No. 4,728,642, issued March 1, 1988 to Pawelchak et al. and U.S. Patent No. 5,006,401, issued April 9, 1991 to Frank, all of which are also owned by the Bristol-Myers Squibb Company of Lawrenceville, New Jersey. Those adhesives are also commercially used in medical products sold by the ConvaTec Division of Bristol-Myers Squibb Company.

Those patents disclose medical grade pressure sensitive compositions including a homogeneous mixture of one or more polyisobutylenes or blends of one or more polyisobutylenes and butyl rubber, one or more styrene radial or block type copolymers, mineral oil, one or more water soluble hydrocolloid gums, and a tackifier. They may also include one or more water swellable cohesive strengthening agents, an anti-oxidant and various other optional ingredients.

Those rubber based hydrocolloid adhesives contain a tackifier, unlike the adhesive material disclosed in the Chen patent. The tackifier is required in the formulation to create the necessary adhesion to the skin. The use of a tackifier can substantially increase adhesion after prolonged skin contact. Because of this adhesive ability, appliances with those adhesives typically need not be removed from the skin for at least three days after attachment to the skin. Such an aggressive nature and the use of a tackifier that does not necessarily support excellent "wet" tack, make those adhesives better suited as a secondary layer, located away from the skin.

Thus, each of the above types pressure sensitive hydrocolloid adhesives have certain qualities that make them excellent for use in medical appliances designed to adhere to the skin and each has drawbacks that make it less desirable for such application. The hydrocolloid adhesive disclosed in the Chen patent is skin friendly, has great initial dry and wet tack, and can be removed from the skin painlessly, and thus is best used where skin contact is required. The hydrocolloid adhesives disclosed in Doyle et al., Pawelchak et al. and Frank patents noted above have reduced wet tack, are more aggressive in short wear periods and are best used to give the appliance enhanced flexibility and comfort, increased moisture tolerance, the ability to swell to create a seal around the stoma in a controlled fashion and can be sterilized without discoloration.

Reference may be made to EP-A-0686381 which discloses the pre-characterizing features of the invention. Reference may also be made to EP-A-0413250, US-A-4538603, US-A-4775374, WO 94/15562 and WO 98/17212.

### SUMMARY OF THE INVENTION

The present invention is defined in the claims.

An advantage of the present invention, in its preferred embodiments, is that it can provide a multi-layered adhesive medical appliance that has the attributes of a wet tack pressure sensitive adhesive for use adjacent the skin, and of a flexible, comfortable, moisture tolerant adhesive that resists degradation after sterilization, and is capable of creating a seal around the stoma in a controlled fashion, for use away from the skin.

A further advantage is that the invention can provide a multi-layered adhesive medical appliance in which different adhesives are utilized such that the advantageous qualities of each type of adhesive are maximized and the disadvantages of each type of adhesive are minimized in order to provide an improved ostomy device or wound care product.

In one preferred embodiment, the second hydrocolloid adhesive is situated between the film layer and the first hydrocolloid adhesive. In a second preferred embodiment, the film layer is situated between the first hydrocolloid adhesive and the second hydrocolloid adhesive.

The appliance has a first surface adapted to adhere to the skin. In both preferred embodiments, the first hydrocolloid adhesive forms the first appliance surface.

The appliance has a second surface. In the first preferred embodiment, the film layer forms the second surface. In the second preferred embodiment, the second hydrocolloid adhesive forms the second appliance surface.

A removable protective layer can be provided adjacent the first appliance surface. The removable protective layer preferably comprises a silicone release coated substrate.

The first hydrocolloid adhesive forms a first adhesive layer. The second hydrocolloid adhesive forms a second adhesive layer. The second adhesive layer is thicker than the first adhesive layer.

The film layer preferably comprises polyethylene or other flexible film. The film layer may have a smooth surface or the surface of the film layer may be embossed.

The first hydrocolloid adhesive includes a polyisobutylene having dispersed therein a water soluble or swellable hydrocolloid or mixture of hydrocolloids.

The second hydrocolloid adhesive includes a homogeneous mixture of one or more polyisobutylenes or blends of one or more polyisobutylenes and butyl rubber, one or more styrene radial or block type copolymers, mineral oil, one or more water soluble hydrocolloid gums, and a tackifier.

The second hydrocolloid adhesive layer preferably also includes one or more swellable cohesive strengthening agents and an anti-oxidant.

### BRIEF DESCRIPTION OF THE DRAWINGS

To these and such other objects that may hereinafter appear, the present invention relates to a multi-layer adhesive medical device as described in the following specification and recited in the annexed claims, taken together with the accompanying drawing, wherein like numerals refer to like parts and in which:
FIG. 1 is an isometric view of the adhesive wafer portion of a conventional ostomy device;
FIG. 2 is a cross-sectional view of the product of Figure 1;
FIG. 3 is a cross-sectional view of the first preferred embodiment of the medical device of the present invention;
FIG. 4 is a cross-sectional view of a second preferred embodiment of the medical appliance of the present invention; and
FIG. 5 is a cross-sectional view of a third preferred embodiment of the medical appliance of the present invention.

### DETAILED DESCRIPTION OF THE DRAWINGS

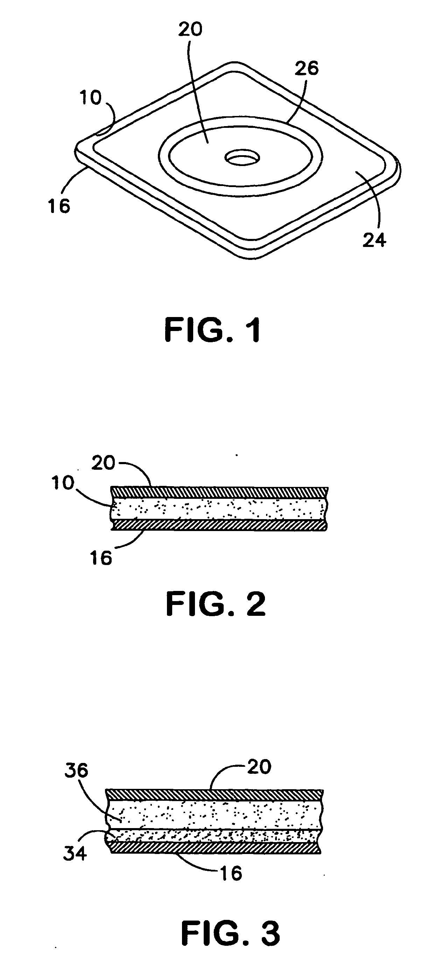

FIG. 1 illustrates a conventional adhesive wafer portion of an ostomy device. As seen in the cross-sectional view of FIG. 2, this wafer consists of a pressure sensitive adhesive layer 10 formed of a single adhesive material. That adhesive material may be, for example, the material disclosed in either the aforementioned Chen patent or that disclosed in any of the aforementioned Doyle et al., Pawelchak et al. or Frank patents. Silicone release paper 16 is situated adjacent the skin facing (bottom) surface of the adhesive layer 10. Polyethylene film 20 is situated on the other (top) surface of the adhesive layer 10.

In a separate process not illustrated and as is well known in the art, see, for example, U.S. Patent Nos. 4,460,363, 4,465,486, 4,419,174, 4,701,169, 4,775,374, 5,951,533, 6,602,232, the single adhesive layer wafer shown in FIG. 1 is attached to a waste collection receptacle such as pouch or bag, either permanently by heat or sonic welding or similar means, or by utilizing inter-engaging coupling rings that permit the collection receptacle to be detached from the wafer. This allows the collection receptacle to be emptied and cleaned, without removing the wafer from the skin, and thereafter to be reattached.

The adhesive wafer is attached to the skin surrounding the stoma after the central opening of the wafer is enlarged to fit around the stoma and release paper 16 is removed from the surface of the adhesive layer. A transparent protective plastic shield 24 may be affixed to film 20 by a ring weld 26, if desired.

The resulting appliance is illustrated in cross-section in FIG. 3. As seen in that figure, the skin friendly first hydrocolloid adhesive layer 34 is preferably substantially thinner than the second hydrocolloid adhesive layer 36.

The resulting product will appear essentially as illustrated in FIG. 3 if the adhesives are extruded into distinct layers 34 and 36.

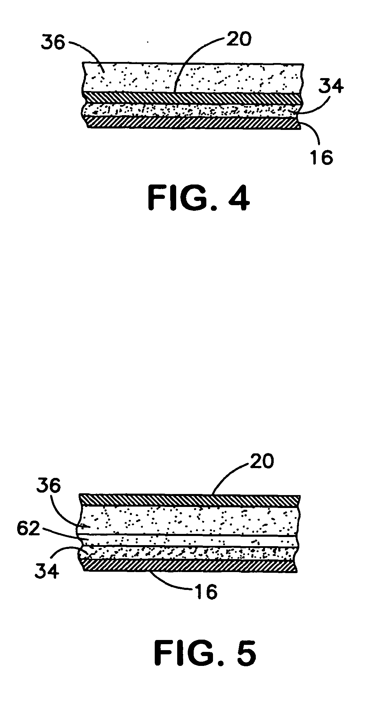

FIG. 4 illustrates the cross-sectional structure of the appliance that includes silicone release film 16, a first hydrocolloid adhesive layer 34, a second hydrocolloid adhesive layer 36 and a film layer 20 situated between the two adhesive layers 34 and 36. A second film layer (not shown) may be situated on top of the second hydrocolloid adhesive layer 36.

FIG. 5 illustrates the cross-sectional embodiment of an appliance having an adhesive layer 62 is situated between the first and second hydrocolloid adhesive layers 34,36. Layer 62 is formed from the mixture of the first hydrocolloid adhesive and the second hydrocolloid adhesive. The relative thicknesses of the adhesive layers 34, 36 and 62 can be adjusted, as required. A film layer 20 is on top of the second hydrocolloid adhesive layer 36.

It is possible that when the second hydrocolloid adhesive layer 36 of the multi-adhesive ostomy embodiments described herein comprises the majority of the adhesive structure at least in the area around the stoma, the adhesive near the stoma is capable of being molded around the stoma so as to form a seal.

Alternatively, it is possible to combine the multi-adhesive hydrocolloid structure described herein with a moldable adhesive such as the type described in U.S. Application No. 10/188,535. The moldable adhesive would be present around the stomal opening and a multi-adhesive portion would surround the moldable adhesive.

It should now be appreciated that the present invention relates to a medical appliance designed to adhere to the skin proximate a stoma or other open wound that has an adhesive layer including two or more different composition pressure sensitive adhesives, each having distinct qualities that make it desirable for use in this type of product. One adhesive is skin friendly, having excellent tack properties and can be removed from the skin painlessly, even after short wear times. The other adhesive is flexible and comfortable to wear. It has high moisture tolerance and the ability to swell to create a seal around the stoma. It has minimal cold flow and will not discolor significantly when sterilized.

## Claims

1. A medical appliance adapted to adhere to the skin comprising a film layer (20), a first hydrocolloid adhesive and a second hydrocolloid adhesive, said second hydrocolloid adhesive having a different composition than said first hydrocolloid adhesive,
wherein said first hydrocolloid adhesive forms a first adhesive layer (34) adapted to adhere to the skin, and said second hydrocolloid adhesive forms a second adhesive layer (36) thicker than said first adhesive layer, and
**characterised in that**:
the first hydrocolloid adhesive has a composition comprising a polyisobutylene having dispersed therein a water soluble or swellable hydrocolloid or mixture of hydrocolloids that provides greater wet tack than the second hydrocolloid adhesive; and
the second hydrocolloid adhesive has a composition comprising a homogeneous mixture of one or more polyisobutylenes or blends of one or more polyisobutylenes and butyl rubber, one or more styrene radial or block type copolymers, mineral oil, one or more water soluble hydrocolloid gums, and a tackifier, the second hydrocolloid adhesive exhibiting, compared to the first hydrocolloid adhesive: (i) greater flexibility; (ii) less cold flow; (iii) greater tolerance to moisture without disintegrating; and (iv) less propensity to discoloration upon sterilization.

2. The medical appliance of Claim 1 wherein said appliance has a first surface adapted to adhere to the skin and wherein said first hydrocolloid adhesive comprises said first appliance surface.

3. The medical appliance of Claim 1 or 2 wherein said second hydrocolloid adhesive is situated between said film layer (20) and said first hydrocolloid adhesive.

4. The medical appliance of Claim 1, 2 or 3, wherein said appliance has a second surface and wherein said film layer (20) comprises said second appliance surface.

5. The medical appliance of any preceding claim, wherein the first and second adhesive layers (34, 36) are in direct contact with each other.

6. The medical appliance of Claim 1 or 2, wherein said film layer (20) is situated between said first hydrocolloid adhesive and said second hydrocolloid adhesive.

7. The medical appliance of any preceding Claim further comprising a removable protective layer (16).

8. The medical appliance of Claim 7, wherein said removable protective layer (16) is situated adjacent said first hydrocolloid adhesive.

9. The medical appliance of Claim 7 or 8, wherein said removable protective layer (16) comprises silicone release material on a substrate.

10. The medical appliance of any preceding Claim, wherein said film layer (20) comprises polyethylene or other flexible material.

11. The medical appliance of Claim 1, wherein said second hydrocolloid adhesive layer further comprises one or more swellable cohesive strengthening agents and an anti-oxidant.

12. The medical appliance of any preceding Claim, wherein said film layer (20) is or can be embossed.

13. The medical appliance of any preceding Claim further comprising a third adhesive layer (62) formed of a mixture of said first hydrocolloid adhesive and said second hydrocolloid adhesive.

14. The medical appliance of Claim 13, wherein said third hydrocolloid adhesive layer (62) is interposed between said first hydrocolloid adhesive layer (34) and said second hydrocolloid adhesive layer (36).

15. The medical appliance of any preceding Claim further comprising a stomal opening.

16. The medical appliance of Claim 15, wherein the second hydrocolloid adhesive has an ability to swell to create a seal around a stoma.

17. The medical appliance of Claim 15 or 16, wherein said second hydrocolloid adhesive comprises a majority of said hydrocolloid adhesives and said adhesives are moldable around said stomal opening.

18. The medical appliance of Claim 15 or 16 further comprising a moldable adhesive portion around said stomal opening, and wherein said first and second hydrocolloid adhesives are arranged around said moldable adhesive portion.

## Patentansprüche

1. Medizinische Vorrichtung zum Kleben auf eine Haut, mit einer Folienschicht (20), einem ersten hydrokolloiden Klebemittel und einem zweiten hydrokolloiden Klebemittel, wobei das zweite hydrokolloide Klebemittel eine andere Zusammensetzung als das erste hydrokolloide Klebemittel hat, und
wobei das erste hydrokolloide Klebemittel eine erste Klebemittelschicht (34) zum Kleben auf die Haut bildet, und das zweite hydrokolloide Klebemittel eine zweite Klebemittelschicht (36) bildet, die dicker als die erste Klebemittelschicht ist,
**dadurch gekennzeichnet, dass**
das erste hydrokolloide Klebemittel eine Zusammensetzung hat, die ein Polyisobutylen aufweist, in dem ein wasserlöslicher/s oder schwellbarer/s Hydrokolloid oder eine Hydrokolloidenmischung enthalten ist, wobei dieser/diese eine größere Feuchteanheftbarkeit als das zweite hydrokolloide Klebemittel aufweist; und
das zweite hydrokolloide Klebemittel eine Zusammensetzung hat, die eine homogene Mischung von einem oder mehreren Poylisobutylenen oder Gemischen von einem oder mehreren Poylisobutylenen und Butylkautschuk, von einem oder mehreren radialartigen oder blockartigen Styren-Copolymeren, Mineralöl, von einem oder mehreren wasserlöslichen hydrokolloiden Gummis, und einem Klebrigmacher hat, wobei das zweite hydrokolloide Klebemittel im Vergleich zum ersten hydrokolloiden Klebemittel aufweist: (i) eine größere Flexibilität,(ii) einen geringeren Kältefluss,(iii) eine größere Toleranz gegenüber Feuchtigkeit ohne Auflösung, und (iv) eine geringere Neigung zu Verfärbung bei Sterilisation.

2. Medizinische Vorrichtung nach Anspruch 1, wobei die Vorrichtung eine erste Fläche zum Kleben auf die Haut hat, und wobei das erste hydrokolloide Klebemittel diese erste Vorrichtungsfläche enthält.

3. Medizinische Vorrichtung nach Anspruch 1 oder 2, wobei das zweite hydrokolloide Klebemittel zwischen der Folienschicht (20) und dem ersten hydrokolloiden Klebemittel angeordnet ist.

4. Medizinische Vorrichtung nach Anspruch 1, 2 oder 3, wobei die Vorrichtung eine zweite Fläche hat und wobei die Folienschicht (20) die zweite Vorrichtungsfläche enthält.

5. Medizinische Vorrichtung nach einem der vorhergehenden Ansprüche, wobei die erste und die zweite Klebemittelschicht (34, 36) im direkten Kontakt miteinander sind.

6. Medizinische Vorrichtung nach Anspruch 1 oder 2, wobei die Folienschicht (20) zwischen dem ersten hydrokolloiden Klebemittel und dem zweiten hydrokolloiden Klebemittel angeordnet ist.

7. Medizinische Vorrichtung nach einem der vorhergehenden Ansprüche, wobei eine entfernbare Schutzschicht (16) vorgesehen ist.

8. Medizinische Vorrichtung nach Anspruch 7, wobei die entfernbare Schutzschicht (16) benachbart zum ersten hydrokolloiden Klebemittel angeordnet ist.

9. Medizinische Vorrichtung nach Anspruch 7 oder 8, wobei die entfernbare Schutzschicht (16) auf einem Substrat ein Silikon-Trennmaterial enthält.

10. Medizinische Vorrichtung nach einem der vorhergehenden Ansprüche, wobei die Folienschicht (20) Polyethylen oder ein anderes flexibles Material enthält.

11. Medizinische Vorrichtung nach Anspruch 1, wobei die zweite hydrokolloide Klebemittelschicht des Weiteren einen oder mehrere quellbare Kohäsionskräfte-stärkende Mittel und ein Anti-Oxidierungsmittel enthält.

12. Medizinische Vorrichtung nach einem der vorhergehenden Ansprüche, wobei die Folienschicht (20) geprägt ist oder geprägt sein kann.

13. Medizinische Vorrichtung nach einem der vorhergehenden Ansprüche, wobei des Weiteren eine dritte Klebemittelschicht (62)
vorgesehen ist, die eine Mischung des ersten hydrokolloiden Klebemittels und des zweiten hydrokolloiden Klebemittels ist.

14. Medizinische Vorrichtung nach Anspruch 13, wobei die dritte hydrokolloide Klebemittelschicht (62) zwischen der ersten hydrokolloiden Klebemittelschicht (34) und der zweiten hydrokolloiden Klebemittelschicht (36) eingefügt ist.

15. Medizinische Vorrichtung nach Anspruch einem der vorhergehenden Ansprüche, wobei des Weiteren eine Atmungsöffnung vorgesehen ist.

16. Medizinische Vorrichtung nach Anspruch 15, wobei das zweite hydrokolloide Klebemittel zur Schaffung einer Dichtung um die Atmungsöffnung anschwellbar ist.

17. Medizinische Vorrichtung nach Anspruch 15 oder 16, wobei das zweite hydrokolloide Klebemittel eine Mehrheit der hydrokolloiden Klebemittel hat, und wobei die Klebemittel formbar um die Atmungsöffnung sind.

18. Medizinische Vorrichtung nach Anspruch 15 oder 16, wobei des Weiteren ein formbarer Klebemittelbereich um die Atmungsöffnung vorgesehen ist, und wobei das erste und zweite hydrokolloide Klebemittel um den formbaren Klebemittelbereich angeordnet sind.

## Revendications

1. Dispositif médical adapté pour adhérer à la peau comprenant une couche de film (20), un premier adhésif hydrocolloïde et un second adhésif hydrocolloïde, ledit second adhésif hydrocolloïde ayant une composition différente dudit premier adhésif hydrocolloïde,
dans lequel ledit premier adhésif hydrocolloïde forme une première couche adhésive (34) aptée pour adhérer à la peau, et ledit deuxième adhésif hydrocolloïde forme une seconde couche adhésive (36) plus épaisse que ladite première couche adhésive, et
**caractérisé en ce que**:
le premier adhésif hydrocolloïde a une composition comprenant un polyisobutylène comprenant dispersé dans celui-ci un hydrocolloïde soluble ou gonflable dans l'eau ou un mélange d'hydrocolloïdes qui assure une plus grande pégosité humide que le second adhésif hydrocolloïde ; et
le second adhésif hydrocolloïde a une composition comprenant un mélange homogène d'un ou plusieurs polyisobutylènes ou des mélanges d'un ou de plusieurs polyisobutylénes et de caoutchouc butyle, d'un ou de plusieurs copolymères de styrènes radiaux ou copolymères à blocs, d'huile minérale, d'une ou de plusieurs gommes hydrocolloïdes solubles dans l'eau, et un agent pégueux, le second adhésif hydrocolloïde présentant, par rapport au premier adhésif hydrocolloïde: (i) une plus grande flexibilité, (ii) moins d'écoulement à froid, (iii) une plus grande tolérance à l'humidité sans se désintégrer, et (iv) une propension moindre à la décoloration lors de la stérilisation.

2. Dispositif médical selon la revendication 1 dans lequel ledit dispositif possède une première surface adaptée pour adhérer à la peau et dans lequel ledit premier adhésif hydrocolloïde comprend ladite première surface du dispositif.

3. Dispositif médical selon la revendication 1 ou 2, dans lequel ledit second adhésif hydrocolloïde est placé entre ladite couche de film (20) et ledit premier adhésif hydrocolloïde.

4. Dispositif médical selon la revendication 1, 2 ou 3, dans lequel ledit dispositif a une seconde surface et dans lequel ladite couche de film (20) comprend ladite seconde surface du dispositif.

5. Dispositif médical selon l'une quelconque des revendications précédentes, dans lequel la première et la seconde couche adhésive (34, 36) sont en contact direct l'une avec l'autre.

6. Dispositif médical selon la revendication 1 ou 2, dans lequel ladite couche de film (20) est située entre ledit premier adhésif hydrocolloïde et ledit second adhésif hydrocolloïde.

7. Dispositif médical selon l'une quelconque des revendications précédentes comprenant en outre une couche protectrice amovible (16).

8. Dispositif médical selon la revendication 7, dans lequel ladite couche de protection amovible (16) est adjacente audit premier hydrocolloïde adhésif.

9. Dispositif médical selon la revendication 7 ou 8, dans lequel ladite couche de protection amovible (16) comprend un matériau de décollement en silicone sur un substrat.

10. Dispositif médical selon l'une quelconque des revendications précédentes, dans lequel ladite couche de film (20) comprend du polyéthylène ou autre matériau flexible.

11. Dispositif médical selon la revendication 1, dans lequel ladite seconde couche adhésive hydrocolloïde comprend en outre un ou plusieurs agents gonflables cohésifs renforçants et un agent anti-axydant.

12. Dispositif médical selon l'une quelconque des revendications précédentes, dans lequel ladite couche de film (20) est ou peut être en relief.

13. Dispositif médical selon l'une quelconque des revendications précédentes comprenant en outre une troisième couche adhésive (62) formée d'un mélange dudit premier adhésif hydrocolloïde et dudit second adhésif hydrocolloïde.

14. Dispositif médical selon la revendication 13, dans lequel ladite troisième couche adhésive hydrocolloïde (62) est interposée entre ladite première couche adhésive hydrocolloïde (34) et ladite seconde couche adhésive hydrocolloïde (36).

15. Dispositif médical selon l'une quelconque des revendications précédentes, comprenant en outre une ouverture stomale.

16. Dispositif médical selon la revendication 15, dans lequel le second adhésif hydrocolloïde a la capacité de gonfler pour former un joint autour d'un abouchement.

17. Dispositif médical selon la revendication 15 ou 16, dans lequel ledit second adhésif hydrocolloïde comprend une majorité desdits adhésifs hydrocolloïdes et lesdits adhésifs sont moulables autour de ladite ouverture stomale.

18. Dispositif médical selon la revendication 15 ou 16 comprenant en outre une partie adhésive moulable autour de ladite ouverture stomale, et dans lequel ledit premier et second adhésif hydrocolloïde sont disposés autour de ladite partie moulable adhésive.
